# EUROPEAN PATENT SPECIFICATION

(11) **EP 3 900 659 B1**
(45) Date of publication and mention of the grant of the patent: **10.06.2026**
(21) Application number: 21169582.0
(22) Date of filing: 21.04.2021
(51) Int. Cl.: A61B 18/14, A61B 18/00

(54) **ABLATION OF A HARD-TO-ACCESS REGION**
ABLATION EINES SCHWER ZUGÄNGLICHEN BEREICHS
ABLATION D'UNE RÉGION DIFFICILE D'ACCÈS

(30) Priority: 22.04.2020 US 202063013957 P; 29.12.2020 US 202063131466 P; 10.03.2021 US 202117197870
(43) Date of publication of application: 27.10.2021
(73) Proprietor: BIOSENSE WEBSTER (ISRAEL) LTD., 2066717 Yokneam (IL)
(72) Inventor: GOVARI, Assaf, Yokneam (IL); BEECKLER, Christopher Thomas, Irvine (US)
(74) Representative: Carpmaels & Ransford LLP

(56) References cited:
- EP-A1- 3 381 396
- WO-A2-02/094115
- US-A- 6 004 269
- US-A1- 2005 015 046
- US-A1- 2015 112 328

## Description

### FIELD OF THE INVENTION

The present invention relates to the ablation of tissue.

### BACKGROUND

The ligament of Marshall (LOM), which is located on the epicardium between the left atrial appendage and the left pulmonary veins, is often a source of paroxysmal atrial fibrillation.

Radio-frequency (RF) ablation and irreversible electroporation (IRE) are leading modalities for ablating cardiac tissue, e.g., for treating atrial fibrillation.

US Patent 9,655,677 describes cardiac tissue ablation catheters including an inflatable and flexible toroidal or spherically shaped balloon disposed at a distal region of an elongate member, a flexible circuit carried by an outer surface of the balloon, the flexible circuit including, a plurality of flexible branches conforming to the radially outer surface of the balloon, each of the plurality of flexible branches including a substrate, a conductive trace carried by the substrate, and an ablation electrode carried by the substrate, the ablation electrode in electrical communication with the conductive trace, and an elongate shaft comprising a guidewire lumen extending in the elongate member and extending from a proximal region of the inflatable balloon to distal region of the inflatable balloon and being disposed within the inflatable balloon, wherein a distal region of the elongate shaft is secured directly or indirectly to the distal region of the inflatable balloon.

EP3381296 A1 discloses a balloon catheter with large area electrodes. It provides a medical apparatus, including a probe having a distal end configured for insertion into a body cavity and containing a lumen that opens through the distal end, and an inflatable balloon deployable through the lumen into the body cavity such that when the balloon is deployed through the lumen and inflated, a distal pole on a distal side of the balloon is located opposite the lumen. The medical apparatus also includes an electrode attached to the distal side of the inflatable balloon and extending over at least 50% of an area of the distal side of the balloon that is within 30° of arc from the distal pole.

US 6,004, 269 discloses an acoustic imaging system for use within a heart, having a catheter, an ultrasound device incorporated into the catheter, and an electrode mounted on the catheter. The ultrasound device directs ultrasonic signals toward an internal structure in the heart to create an ultrasonic image, and the electrode is arranged for electrical contact with the internal structure. A chemical ablation device mounted on the catheter ablates at least a portion of the internal structure by delivery of fluid to the internal structure. The ablation device may include a material that vibrates in response to electrical excitation, the ablation being at least assisted by vibration of the material. The ablation device may alternatively be a transducer incorporated into the catheter, arranged to convert electrical signals into radiation and to direct the radiation toward the internal structure. The electrode may be a sonolucent structure incorporated into the catheter. In one embodiment there is provided an electrophysiological heart catheter having an expansible balloon on a deflectable portion. Two axially spaced-apart conductive stripes are applied to the balloon surface.

### SUMMARY OF THE INVENTION

There is provided, in accordance with the present invention, an apparatus as claimed in claim 1 hereinafter. Further embodiments are provided in the dependent claims.

The present invention will be more fully understood from the following detailed description of embodiments thereof, taken together with the drawings, in which:

### BRIEF DESCRIPTION OF THE DRAWINGS

Fig. 1 is a schematic, pictorial illustration of a medical system;
Fig. 2 is a schematic pictorial illustration of elements of a medical probe used in the system;
Fig. 3 is a flowchart of unclaimed steps used for ablation of tissue using the medical probe;
Figs. 4A - 4D are schematic illustrations of some of the steps of the flowchart; and
Fig. 5 is a schematic illustration of a medical probe, in accordance with some embodiments of the present invention.

### DETAILED DESCRIPTION OF EMBODIMENTS

### OVERVIEW

Many regions of the heart are accessed endocardially relatively easily, and so are amenable to ablation by conventional catheters, such as a focal catheter or a balloon catheter. However, there are certain regions of the heart, such as the ligament of Marshall, that are difficult to access endocardially, typically because a possible endocardial path is tortuous, comprising one or more relatively acute angular bends that a conventional catheter is unable to traverse. While these regions may in some cases be accessed epicardially, endocardial access is preferred.

To address this challenge, the inventors have developed apparatuses and methods for endocardially accessing difficult-to-reach regions of a patient's anatomy, and the description herein is provided by way of example for one such region, the ligament of Marshall. The description may be modified, *mutatis mutandis,* for other difficult-to-reach regions.

A medical probe comprises three elements: a flexible guidewire, a tube that is configured to thread over the guidewire, and a shaft, having one or more (typically, two or more) expandable electrodes, at a distal end of the shaft, that may traverse the tube. In contrast to the conventional catheters referred to above, which cannot access the difficult-to-reach regions, the probe is able to access the regions, by virtue of its narrow width and flexibility.

The guidewire is navigated to a region targeted for ablation, and then the tube of the probe is threaded over the guidewire, until a distal end of the tube is in proximity to the target region. The shaft with its expandable electrodes is then pushed into the tube, and is pushed until the expandable electrodes exit the tube distal end, at which point the electrodes expand such that at least one of the electrodes contacts the target region. (Thus, the tube guides the expandable electrodes along the desired path to the target region.) Subsequently, the expanded electrodes may be used to ablate the target region, typically by passing bipolar electrical current between the electrodes.

Advantageously, the electrodes are large enough to transfer electrical current for ablation without being irreparably damaged. In addition, the shaft is large enough to support conductors that are undamaged by the electrical current transfer.

In some embodiments, the electrodes are self-expanding by virtue of being formed from a shape-memory material. In other embodiments, the electrodes are actively expanded. For example, an inflatable balloon may be coupled to the distal end of the shaft, the surface of the balloon being coated with a suitable metallic material so as to define two electrodes. To expand the electrodes, the balloon may be inflated.

### SYSTEM DESCRIPTION

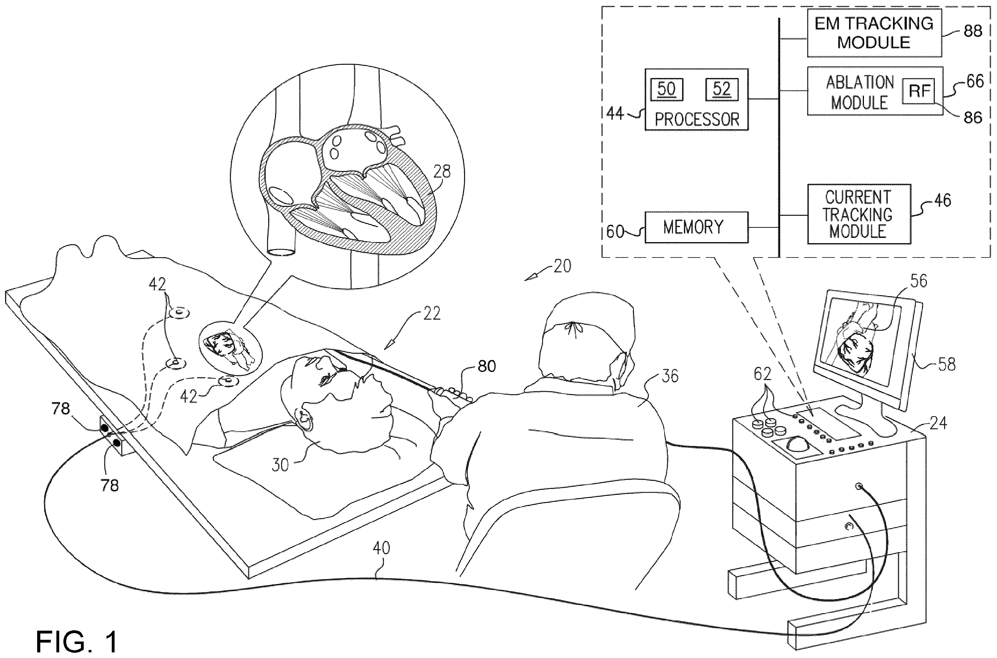

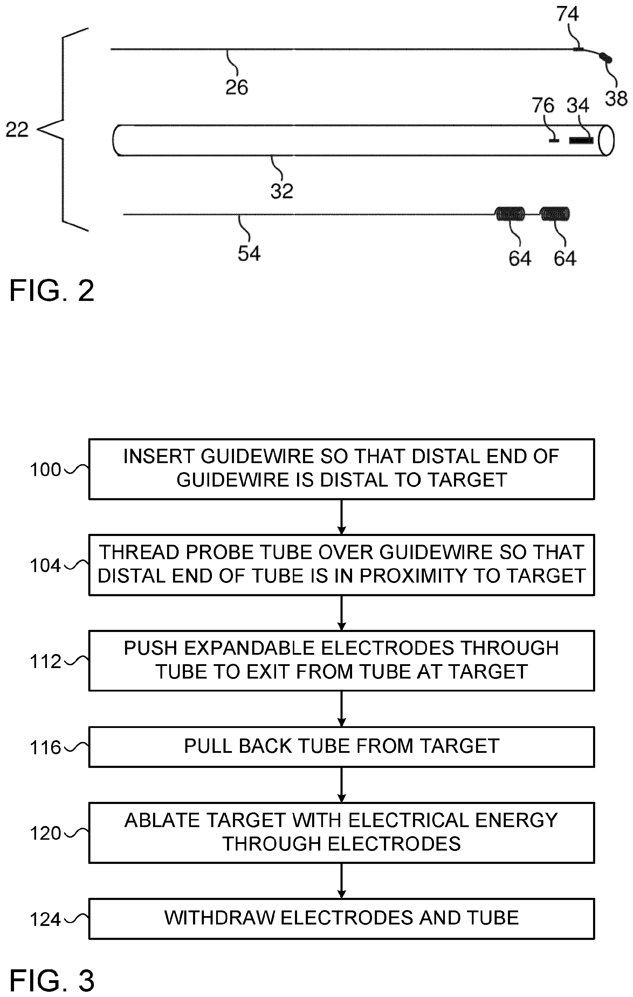

Fig. 1 is a schematic, pictorial illustration of a medical system 20 comprising a medical probe 22 and a control console 24, and Fig. 2 is a schematic pictorial illustration of elements of the medical probe. Medical system 20 may be based, for example, on the CARTO^{®} system, produced by Biosense Webster Inc. of 31 Technology Drive, Irvine, CA 92618 USA. Probe 22 is used as a catheter, and is also referred to herein as catheter 22.

Fig. 1 illustrates a physician 36 using a handle 80 to control probe 22. In embodiments described hereinbelow, probe 22 is used for ablation of tissue in a heart 28 of a patient 30 (also referred to herein as a subject). Typically, probe 22 is used to ablate elements of the heart that are difficult to access, and by way of example, ablation of a portion of a ligament of Marshall of heart 28 is described herein. However, it will be understood that medical probe 22 may be used, *mutatis mutandis,* for other therapeutic and/or diagnostic purposes in the heart or in other body organs.

As shown in Fig. 2, probe 22 comprises a flexible guidewire 26, a flexible tube 32, and a flexible shaft 54.

Guidewire 26 is typically bent at its distal end, as illustrated, and typically comprises a location sensor 38, comprising at least one coil, at the distal end. Alternatively or additionally, guidewire 26 typically has at least one electrode 74, at its distal end, which may also be used for sensing location. In one embodiment guidewire 26 is formed as a stainless steel coil having a diameter of approximately 325 microns, and conductors for sensor 38 and/or electrode 74 traverse a lumen of the coil.

Flexible tube 32 typically also has a location sensor 34, comprising at least one coil, at its distal end. Alternatively or additionally, tube 32 typically has at least one electrode 76, at its distal end, which may be used for sensing location. Tube 32 is dimensioned to have an internal diameter permitting it to thread over guidewire 26, and an external diameter of approximately 1 mm, permitting it to enter a vein or other body of the order of 1 mm in diameter. Tube 32 may be constructed from any stable biocompatible plastic which may be formed with these dimensions. Conductors for sensor 34 and/or electrode 76 may be formed within a wall of tube 32.

Flexible shaft 54 is typically formed as a braided tube (made of polyimide, for example), the braiding resisting any tendency of the shaft to kink. At the distal end of the shaft are one or more (typically, two or more) expandable electrodes 64. Electrodes 64 are configured to be large enough to convey ablation energy to tissue that contacts the electrodes, without the electrodes permanently deforming. Similarly, conductors connected to the electrodes, configured to convey the ablation energy, traverse the shaft and are dimensioned to be large enough to convey the ablation energy without permanent deformation.

Each of electrodes 64 is described herein as being "expandable," in that the electrode may expand, upon exiting tube 32, from a radially-compressed configuration, which the electrode assumes while inside of tube 32, to a radially-expanded configuration. In some embodiments, each electrode is formed from a shape-memory material such as Nitinol (optionally coated with a biocompatible material such as gold), such that the electrode expands due to the shape-memory effect, i.e., the electrode is self-expanding. In other embodiments, the electrodes are actively expanded by the application of electrical and/or mechanical energy. Such energy may be applied by physician 36 or by processor 44 (described below), optionally in response to an input from the physician. In the context of the present application, including the claims, the term "expandable electrode" includes both a self-expanding electrode and an electrode that is not self-expanding.

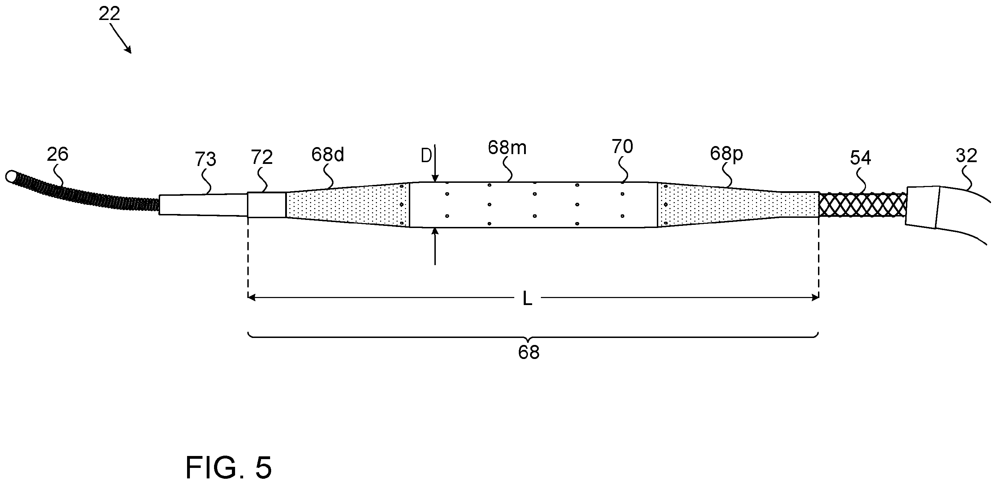

In general, each of electrodes 64 may have any suitable form. For example, as shown in Fig. 2, each electrode may have a helical or helicoid configuration. Alternatively, each electrode may comprise a basket of circumferentially-distributed spines disposed over the shaft. In such embodiments, the electrode may be self-expanding, or the expansion may be effected by the pulling of a pull wire coupled to the basket so as to retract the distal end of the basket towards the proximal end of the basket. In yet other embodiments, as described below with reference to Fig. 5, each of electrodes 64 comprises a coated portion of an inflatable balloon, such that the electrodes expand upon inflation of the balloon.

In the configuration shown in Figure 1, a control console 24 is connected, by a cable 40, to body surface electrodes, which typically comprise adhesive skin patches 42 that are affixed to patient 30. Control console 24 also comprises a processor 44 which is coupled to a number of modules, the modules comprising software and/or hardware components. Details and functionality of the modules are described below.

Processor 44 determines location coordinates, i.e., position coordinates and orientation coordinates, of the distal ends of guidewire 26 and tube 32 based on signals received respectively from sensors 38 and 34 by an electromagnetic (EM) tracking module 88. The sensors generate their signals in response to magnetic fields, transmitted by alternating magnetic field radiators 78 positioned beneath patient 30, traversing the sensors.

Alternatively or additionally, processor 44, in conjunction with a current tracking module 46, determines location coordinates of the distal ends of guidewire 26 and/or tube 32 inside heart 28 based on impedances and/or currents measured between adhesive skin patches 42 and electrodes 74 and 76. Alternatively or additionally to being used as location sensors during a medical procedure, electrodes 74 and 76 may perform other tasks such as measuring electrical activity of heart 28.

Processor 44 may comprise real-time noise reduction circuitry 50 typically configured as a field programmable gate array (FPGA), followed by an analog-to-digital (A/D) signal conversion integrated circuit 52. The processor can pass the signals from A/D circuit 52 to another processor and/or can be programmed to determine the location coordinates referred to above.

Impedance and current-based location tracking techniques are described, for example, in U.S. Patents 5,983,126, 6,456,864, and 5,944,022. Electromagnetic location tracking techniques are described, for example, in U.S. Patents 5,391,199, 6,690,963, and 6,892,091. The methods of location sensing described hereinabove are implemented in the above-mentioned CARTO^{®} system and are described in detail in the patents cited above.

Prior to insertion of elements of probe 22 into patient 30, processor 44 acquires an electroanatomical map 56 of heart 28. Typically data for the map is acquired using a probe other than probe 22, such as a focal catheter which is configured to be both tracked by module 46 and to acquire signals from regions of heart chambers contacted by the catheter. Typically, although not necessarily, processor 44 uses the signals to determine local activation times (LATs) of the heart chambers, and incorporates the LATs into map 56. Map 56 is stored in a memory 60 accessible by processor 44, and during the procedure the processor can present map 56 to physician 36 on a display 58.

During the procedure using probe 22, processor 44 may overlay an icon, representing the location of the distal ends referred to above, on map 56, so enabling physician 36 to track the distal ends.

Memory 60 may comprise any suitable volatile and/or nonvolatile memory, such as random access memory or a hard disk drive. In some embodiments, physician 36 can manipulate map 56 using one or more input devices 62. In alternative embodiments, display 58 may comprise a touchscreen that can be configured to accept inputs from physician 36, in addition to presenting map 56.

Control console 24 also comprises an ablation module 66. Ablation module 66 is configured to monitor and control ablation parameters such as the level and the duration of ablation power (e.g., radio-frequency (RF) energy) conveyed to electrodes 64 from ablation module 66, and module 66 typically comprises a generator 86, such as an RF generator, for this purpose.

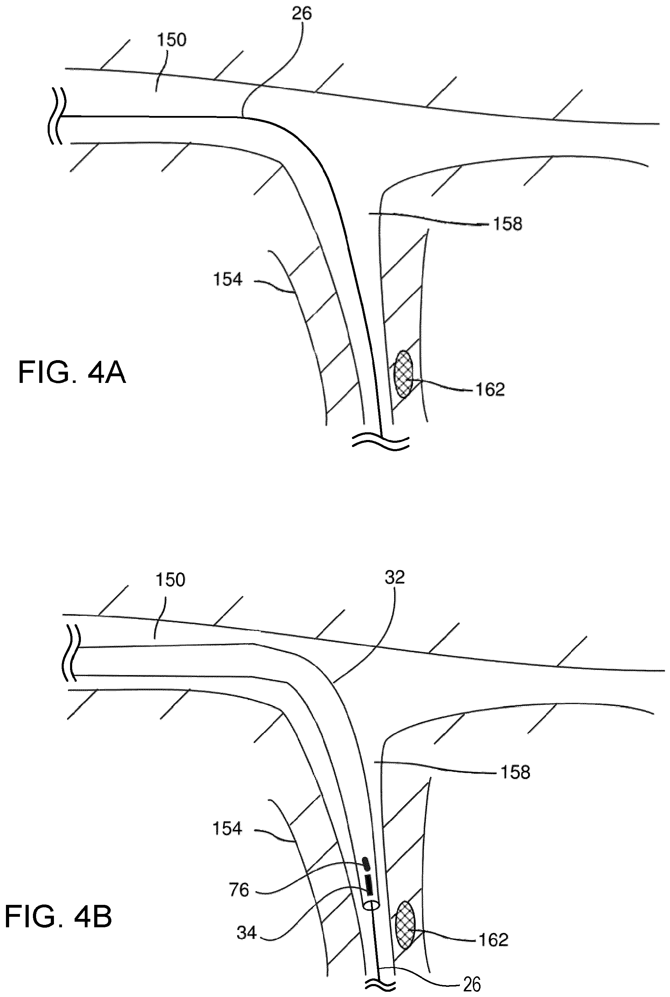

Fig. 3 is a flowchart of example, unclaimed, steps used for ablation of tissue using probe 22, and Figs. 4A - 4D are schematic illustrations of some of the steps. Figs. 4A - 4D schematically illustrate a coronary sinus 150 of heart 28, a ligament of Marshall (LOM) 154 connected to the coronary sinus, and a vein 158 through the LOM. The following description assumes, by way of example, that a target region 162 of LOM 154 is to be ablated.

In an initial step 100, physician 36 inserts guidewire 26 into the subject, and then navigates the guidewire into heart 28. Within the heart, the guidewire is navigated via coronary sinus 150 into ligament of Marshall vein 158 until a distal end of the guidewire is distal to target region 162 (e.g., within 10 mm of the target region). The physician is typically assisted in the navigation by processor 44 using signals from sensor 38, or from electrode 74, to display an icon representing the location of the distal end of the guidewire on map 56. Alternatively or additionally physician 36 may use fluoroscopy to perform the navigation.

It will be understood that the intra-cardiac navigation to a difficult to access site such as LOM 154 is complicated, since guidewire 26 has to bend around one or more acute angles. For example, to reach the LOM, the guidewire may need to pass through the inferior vena cava, right atrium, and coronary sinus. The complicated navigation is facilitated by guidewire 26 being configured to be extremely thin yet constructed to be flexible without kinking.

The final stage of step 100 is illustrated in Fig. 4A, showing guidewire 26 within LOM vein 158.

Once physician 36 has satisfactorily navigated the distal end of the guidewire to be distal to target region 162, in a tube threading step 104 the physician threads tube 32 over the guidewire, and continues the threading until a distal end of the tube is in proximity to target region 162. To assist the physician in correctly locating the distal end of the tube, processor 44 may use signals from sensor 34 or from electrode 76 to display a representation of the tube distal end on map 56. Alternatively or additionally, the physician may use fluoroscopy to correctly locate the tube distal end.

The final stage of step 104 is illustrated in Fig. 4B, showing the distal end of tube 32 near target region 162.

In an electrode traversal step 112, the physician inserts shaft 54, with at least one expandable electrode 64 connected to the shaft distal end, into tube 32. While electrodes 64 may be compressed by the walls of the tube, they are still able to traverse the tube when the physician pushes on a proximal end of the shaft.

The physician continues to push on the proximal end of the shaft, so continuing the traversal of electrodes 64 through tube 32, until the electrodes exit the distal end of the tube. On exiting the tube distal end the electrodes may self-expand such that at least one of the electrodes contacts target region 162. In other words, provided the electrodes are self-expanding, the physician may cause the electrodes to expand distally to the distal end of tube 32 simply by pushing the electrodes from the distal end of the tube. In the event that the electrodes are not self-expanding, the electrodes may be expanded by performing an additional, electrode-expanding step, comprising, for example, the inflation of a balloon (Fig. 5). The electrode-expanding step may be performed between electrode traversal step 112 and first withdrawal step 116 (described below), or between first withdrawal step 116 and ablation step 120.

In an example the location of electrodes 64 relative to target region 162 may be verified by current tracking module 47 using impedances and/or currents between the electrodes and patches 42. Alternatively or additionally, the location of the electrodes may be verified fluoroscopically.

In a first withdrawal step 116, once electrodes 64 have exited the tube distal end and at least one of the electrodes is in contact with target region 162, the physician partially withdraws tube 32, typically by a withdrawal of approximately 1 cm, so that its distal end is no longer close to the target region.

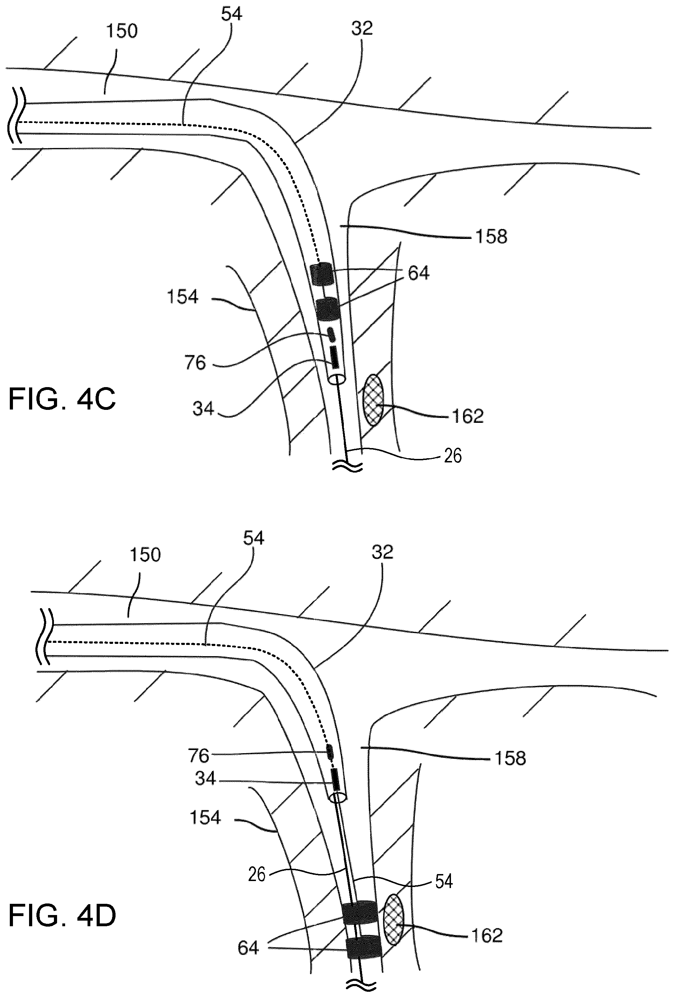

Fig. 4C illustrates shaft 54 and expandable electrodes 64 within tube 32, before the electrodes exit the distal end of the tube. Fig. 4D illustrates the state at the conclusion of step 116, i.e., when electrodes 64 have exited the distal end of tube 32, and have expanded to contact target region 162, and when the distal end of the tube has been partially withdrawn.

In an ablation step 120 the physician operates processor 44 and ablation module 66 to supply electrical current to electrodes 64. If there are two or more electrodes 64, then the supplied current may be bipolar, i.e., the current may be passed between the electrodes so as to convey ablation energy to the tissue. Alternatively (e.g., if there is only one electrode 64), the supplied ablation energy may be unipolar, i.e., an electrical current may be applied between one of electrodes 64 and a return electrode (not shown) connected to generator 86. The return electrode may be disposed outside the body of patient 30; for example, the return electrode may comprise a patch coupled to the patient's body.

In some examples, RF electrical current is supplied to the electrodes, such that RF ablation of the tissue is performed. Alternatively, pulsed current may be supplied so as to perform irreversible electroporation (IRE) or pulsed field ablation (PFA).

In a second withdrawal step 124, once the physician completes the ablation in step 120, the physician may advance the tube to the electrodes, withdraw electrodes 64 into the tube, then withdraw the combination of the tube, shaft 54, and electrodes 64 from patient 30. In some examples, prior to withdrawing the electrodes into the tube, the electrodes may be compressed, e.g., by deflating a balloon (Fig. 5). Subsequently, the guidewire may also be withdrawn.

The flowchart of Fig. 3 describes the ablation of one target region. However, examples of the disclosure are not limited to ablation of a single region, but rather may be used to ablate two or more separate regions during a single ablation procedure. For example, if there is a second target region, closer to the coronary sinus than target region 162, the electrodes may be withdrawn into the tube as described in step 124, the combination may be moved into proximity with the second region, and the electrodes may be pushed to exit from the tube distal end and expand to contact the second region in preparation for ablation of that region.

Reference is now made to Fig. 5, which is a schematic illustration of probe 22, in accordance with some embodiments of the present invention.

Probe 22 comprises an inflatable balloon 68 coupled to the distal end of shaft 54. Balloon 68 comprises an electrically-conducting proximal portion 68p and an electrically-conducting distal portion 68d, along with an electrically-insulating middle portion 68m that insulates proximal portion 68p from distal portion 68d. (Typically, there are no electrodes or any other electrically-conducting elements disposed on electrically-insulating middle portion 68m.) The proximal and distal ends of the balloon - i.e., the proximal end of proximal portion 68p and the distal end of distal portion 68d - are bonded to the shaft.

Balloon 68 is typically made from a polymer, such as polyurethane, with each of the proximal and distal portions of the balloon additionally comprising an electrically-conducting metallic coating (comprising gold, for example) that coats the polymer. (The coating is represented in Fig. 5 by a dotted hatch pattern.) Conductors (e.g., wires) traversing the shaft connect these metallic coatings to generator 86 (Fig. 1).

To coat the proximal and distal portions of the polymer, the polymer may be placed into a plating bath with middle portion 68m masked. To facilitate the plating, an electrically-charged seed layer (comprising, for example, silver, palladium, titanium tungsten, and/or titanium) may be deposited onto the polymer prior to the plating.

Typically, for increased contact with the target region, each of the proximal and distal portions of the balloon is electrically-conducting over at least half of its circumference. For example, the aforementioned electrically-conducting metallic coating may extend around the entire circumference of each of these portions. Alternatively, at least one of these portions may comprise multiple discrete electrodes that collectively cover at least half of the circumference of the portion. As a purely illustrative example, two electrodes may each span 150 degrees, with two 30-degree electrically-insulative gaps separating the electrodes from one another. Such electrodes may be formed, for example, by performing the coating procedure outlined above with the placement of additional masks over the inter-electrode gaps.

Typically, balloon 68 is relatively elongated, so as to facilitate contacting target region 162 while disposed within a narrow lumen such as vein 158 (Figs. 4A-D). The length L of the balloon is 2-20 times greater than the maximal cross-sectional diameter D of the balloon, which is between 1 and 3 mm. (Typically, D is the diameter of middle portion 68m, or at least of the axial center thereof. For example, middle portion 68m may be of a constant diameter D, with the proximal and distal portions of the balloon being of a variable diameter such that the balloon reaches its minimal diameter at the proximal and distal ends thereof.)

In some embodiments, sensing electrodes and/or other sensors are coupled to middle portion 68m and are connected to console 24 (Fig. 1) via conductors (e.g., wires) traversing the shaft. Such sensors may be used, for example, to acquire electrophysiological signals from tissue or to measure impedance.

In some embodiments, the balloon further comprises an atraumatic tip 72 disposed distally to the electrically-conducting distal portion. Atraumatic tip 72 is typically made from a relatively soft and compressible material such as polyurethane or polyether block amide (PEBA).

In some embodiments, as shown in Fig. 5, the distal end of the shaft protrudes from the balloon. The distal end of the shaft may comprise an atraumatic tip 73, which may be made from polyurethane, PEBA, or any other suitable material. In other embodiments - particularly those in which the balloon comprises atraumatic tip 72 - the shaft may terminate proximally to the distal end of the balloon.

The embodiment of probe 22 shown in Fig. 5 is typically used as described above with reference to Fig. 3, with the electrically-conducting proximal and distal portions of the balloon functioning as expandable electrodes 64 (Fig. 2). Typically, electric current is passed between the two electrically-conducting portions of the balloon, i.e., the ablation is bipolar.

Typically, the balloon is shaped to define multiple apertures 70 passing through the wall of the balloon. A fluid-delivery tube (not shown), configured to deliver a fluid, such as saline, from a pump in console 24 (Fig. 1) to the interior of the balloon, passes through shaft 54. To inflate the balloon (and subsequently keep the balloon inflated during the ablation procedure), the fluid may be streamed through the balloon via the fluid-delivery tube. The flow of the fluid through apertures 70 may also help transfer heat from the tissue and prevent coagulation of the patient's blood while electrical energy is conveyed to the tissue.

It will be appreciated by persons skilled in the art that the present invention is not limited to what has been particularly shown and described hereinabove. Rather, the scope of the present invention is defined by the claims and includes both combinations and subcombinations of the various features described hereinabove which fall within the scope of the claims, as well as variations and modifications thereof falling within the scope of the claims which would occur to persons skilled in the art upon reading the foregoing description.

## Claims

1. Apparatus (22), comprising:
a shaft (24); and
an inflatable balloon (68) coupled to a distal end of the shaft, the balloon comprising:
a proximal portion (68p) that is electrically-conducting over at least half of a proximal-portion circumference of the proximal portion;
a distal portion (68d) that is electrically-conducting over at least half of a distal-portion circumference of the distal portion; and
an electrically-insulating middle portion (68m) that insulates the proximal portion from the distal portion, and
**characterized in that** a length of the balloon is 2-20 times greater than a maximal cross-sectional diameter of the balloon, the maximal cross-sectional diameter of the balloon being between 1 and 3 mm.

2. The apparatus according to claim 1, wherein the balloon is shaped to define multiple apertures (70) passing through a wall of the balloon.

3. The apparatus according to claim 1, wherein the balloon further comprises an atraumatic tip (72) disposed distally to the electrically-conducting distal portion.

4. The apparatus according to claim 1, wherein the distal end of the shaft protrudes from the balloon.

5. The apparatus according to claim 1, wherein no electrically-conducting element is disposed on the electrically-insulating middle portion.

## Patentansprüche

1. Einrichtung (22), umfassend:
einen Schaft (24); und
einen aufblasbaren Ballon (68), der mit einem distalen Ende des Schafts gekoppelt ist, der Ballon umfassend:
einen proximalen Abschnitt (68p), der über mindestens eine Hälfte eines Proximalabschnittsumfangs des proximalen Abschnitts elektrisch leitend ist;
einen distalen Abschnitt (68d), der über mindestens eine Hälfte eines Distalabschnittsumfangs des distalen Abschnitts elektrisch leitend ist; und
einen elektrisch isolierenden Mittelabschnitt (68m), der den proximalen Abschnitt von dem distalen Abschnitt isoliert, und
**dadurch gekennzeichnet, dass** eine Länge des Ballons 2-20 mal größer als ein maximaler Querschnittsdurchmesser des Ballons ist, wobei der maximale Querschnittsdurchmesser des Ballons zwischen 1 und 3 mm liegt.

2. Einrichtung nach Anspruch 1, wobei der Ballon geformt ist, um mehrere Öffnungen (70) zu definieren, die durch eine Wand des Ballons hindurchgehen.

3. Einrichtung nach Anspruch 1, wobei der Ballon ferner eine atraumatische Spitze (72) umfasst, die distal zu dem elektrisch leitenden distalen Abschnitt angeordnet ist.

4. Einrichtung nach Anspruch 1, wobei das distale Ende des Schafts aus dem Ballon hervorsteht.

5. Einrichtung nach Anspruch 1, wobei kein elektrisch leitendes Element auf dem elektrisch isolierenden Mittelabschnitt angeordnet ist.

## Revendications

1. Appareil (22), comprenant :
une tige (24) ; et
un ballonnet gonflable (68) accouplé à une extrémité distale de la tige, le ballonnet comprenant :
une partie proximale (68p) qui est électriquement conductrice sur au moins la moitié d'une circonférence de partie proximale de la partie proximale ;
une partie distale (68d) qui est électriquement conductrice sur au moins la moitié d'une circonférence de partie distale de la partie distale ; et
une partie centrale électriquement isolante (68m) qui isole la partie proximale de la partie distale, et
**caractérisé en ce qu'**une longueur du ballonnet est de 2 à 20 fois supérieure au diamètre maximal de la section transversale du ballonnet, le diamètre maximal de la section transversale du ballonnet étant compris entre 1 et 3 mm.

2. Appareil selon la revendication 1, dans lequel le ballonnet est formé pour définir de multiples ouvertures (70) passant à travers une paroi du ballonnet.

3. Appareil selon la revendication 1, dans lequel le ballonnet comprend en outre un embout atraumatique (72) disposé distalement par rapport à la partie distale électriquement conductrice.

4. Appareil selon la revendication 1, dans lequel l'extrémité distale de la tige fait saillie depuis le ballonnet.

5. Appareil selon la revendication 1, dans lequel aucun élément électriquement conducteur n'est disposé sur la partie centrale électriquement isolante.
